# EUROPEAN PATENT SPECIFICATION

(11) **EP 3 786 840 B1**
(45) Date of publication and mention of the grant of the patent: **08.04.2026**
(21) Application number: 20191395.1
(22) Date of filing: 17.08.2020
(51) Int. Cl.: G06V 10/764, G06V 10/82, G06V 40/10, G06T 7/70, G06T 7/00, A61B 8/00

(54) **INFORMATION PROCESSING APPARATUS, INSPECTION SYSTEM, INFORMATION PROCESSING METHOD, AND STORAGE MEDIUM THAT ARE USED IN A DIAGNOSIS BASED ON A MEDICAL IMAGE**
INFORMATIONSVERARBEITUNGSVORRICHTUNG, INSPEKTIONSSYSTEM, INFORMATIONSVERARBEITUNGSVERFAHREN UND SPEICHERMEDIUM, DIE IN EINER DIAGNOSE BASIEREND AUF EINEM MEDIZINISCHEN BILD VERWENDET WERDEN
APPAREIL DE TRAITEMENT D'INFORMATIONS, SYSTÈME D'INSPECTION, PROCÉDÉ DE TRAITEMENT D'INFORMATIONS ET SUPPORT D'INFORMATIONS UTILISÉS DANS UN DIAGNOSTIC BASÉ SUR UNE IMAGE MÉDICALE

(30) Priority: 26.08.2019 JP 2019154069
(43) Date of publication of application: 03.03.2021
(73) Proprietor: CANON KABUSHIKI KAISHA, OHTA-KU Tokyo 146-8501 (JP)
(72) Inventor: KOTOKU, Masashi, Ohta-ku, Tokyo 146-8501 (JP); ODAGIRI, Jun, Ohta-ku, Tokyo 146-8501 (JP)
(74) Representative: Canon Europe Limited

(56) References cited:
- JP-A- 2018 175 007
- US-A1- 2015 031 990
- US-A1- 2016 174 934
- US-A1- 2017 215 842
- US-A1- 2017 360 403
- US-A1- 2018 228 460
- ZHE CAO ET AL: "OpenPose: Realtime Multi-Person 2D Pose Estimation using Part Affinity Fields", ARXIV.ORG, CORNELL UNIVERSITY LIBRARY, 201 OLIN LIBRARY CORNELL UNIVERSITY ITHACA, NY 14853, 18 December 2018 (2018-12-18), XP080994611
- VINKLE SRIVASTAV ET AL: "MVOR: A Multi-view RGB-D Operating Room Dataset for 2D and 3D Human Pose Estimation", ARXIV.ORG, CORNELL UNIVERSITY LIBRARY, 201 OLIN LIBRARY CORNELL UNIVERSITY ITHACA, NY 14853, 24 August 2018 (2018-08-24), XP081502482

## Description

### BACKGROUND OF THE INVENTION

### Field of the Invention

The present invention relates to an information processing apparatus, an inspection system, an information processing method, and a storage medium that are used in a diagnosis based on a medical image in the medical field.

### Description of the Related Art

In the medical field, a doctor makes diagnoses using medical images captured by various modalities (inspection systems). Examples of the modalities include an ultrasound diagnosis apparatus and a photoacoustic tomography apparatus (hereinafter referred to as a "PAT apparatus"). Examples of the modalities also include a magnetic resonance imaging apparatus (hereinafter referred to as an "MRI apparatus") and a computed tomography apparatus (hereinafter referred to as an "X-ray CT apparatus").

Japanese Patent Laid-Open No. 2018-175007 discusses a system that, based on the positional relationship between an inspection system and a subject, distinguishes (identifies) which part of the subject is captured to obtain the medical images used in these diagnoses.

Specifically, based on an external appearance image obtained by capturing a subject under inspection and a probe, the positions of the subject and the probe are identified by template matching with template images of the subject and the probe, and an inspection part is calculated from the positional relationship between the subject and the probe.

However, image recognition based on the template matching discussed in Japanese Patent Laid-Open No. 2018-175007 can only deal with limited environments and conditions indicated by template images in which the position of a camera and the positions and orientations of a patient and a probe are stored.

US 2018/228460 A1 discusses estimating patient internal anatomical structures from surface data. A method for controlling a scanner comprises: sensing an outer surface of a body of a subject to collect body surface data, using machine learning to predict a surface of an internal organ of the subject based on the body surface data, and controlling the scanner based on the predicted surface of the internal organ.

### SUMMARY OF THE INVENTION

The present disclosure has been made in consideration of the aforementioned issues, and realizes an information processing apparatus, an inspection system, and an information processing method that enable the identification of an inspection part of a subject with higher accuracy in an inspection by an inspection system.

The present invention in its first aspect provides an information processing apparatus as specified claims 1 to 8.

The present invention in its second aspect provides an inspection system as specified claim 9.

The present invention in its third aspect provides an information processing method as specified claim 10.

The present invention in its fourth aspect provides a computer-readable storage medium as specified claim 11.

According to the present invention, it is possible to identify an inspection part of a subject with higher accuracy in an inspection by an inspection system.

Further features of the present invention will become apparent from the following description of exemplary embodiments with reference to the attached drawings. Each of the embodiments of the present invention described below can be implemented solely or as a combination of a plurality of the embodiments or features thereof where necessary or where the combination of elements or features from individual embodiments in a single embodiment is beneficial.

### BRIEF DESCRIPTION OF THE DRAWINGS

Fig. 1 is a diagram illustrating an example of a configuration of an inspection system according to a first embodiment.
Fig. 2 is a diagram illustrating an example of a configuration of a probe according to the first embodiment.
Fig. 3 is a block diagram illustrating an example of the configuration of the inspection system according to the first embodiment.
Fig. 4 is a flowchart illustrating an entire flow of the inspection system according to the first embodiment.
Fig. 5 is a flowchart illustrating a processing flow of a measurement process according to the first embodiment.
Fig. 6 is a flowchart illustrating a processing flow of ultrasound image processing according to the first embodiment.
Fig. 7 is a flowchart illustrating a processing flow of the measurement process according to a first variation of the first embodiment.
Fig. 8 is a flowchart illustrating a processing flow of a human body position/orientation prediction process according to the first embodiment.
Fig. 9 is a flowchart illustrating a processing flow of inspection part identification A according to the first embodiment.
Fig. 10 is a flowchart illustrating a processing flow of measurement post-processing according to the first embodiment.
Fig. 11 is a flowchart illustrating a processing flow of the measurement process according to a second variation of the first embodiment.
Fig. 12 is a flowchart illustrating a processing flow of inspection part identification B according to the first embodiment.
Fig. 13 is a flowchart illustrating a processing flow of the inspection part identification B according to a third variation of the first embodiment.
Fig. 14 is an image diagram of a measurement using the inspection system according to the first embodiment.
Figs. 15A to 15D are image diagrams of display output results obtained when a measurement is made using the inspection system according to the first embodiment.
Fig. 16 is an image diagram illustrating an external appearance image and markers attached to the probe within the external appearance image according to the first embodiment.

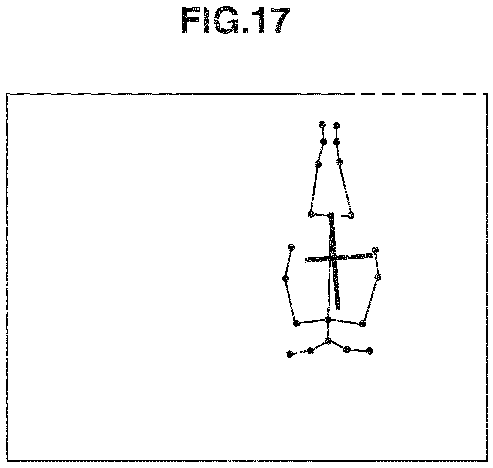

Fig. 17 is an image diagram in which skeleton information as a prediction result of a position and an orientation of a human body and cross lines as a prediction result of a position and an orientation of the probe are superimposed on each other according to the first embodiment.
Fig. 18 is an image diagram of a screen displayed on a display when an ultrasound image is measured according to the first embodiment.
Fig. 19 is an image diagram of a screen displayed on the display when an inspection part is identified after the ultrasound image is finalized according to the first embodiment.
Fig. 20 is an image diagram illustrating a state of an inspection according to a fourth variation of the first embodiment.
Figs. 21A to 21C are image diagrams of display output results obtained when a measurement is made using an inspection system according to the fourth variation of the first embodiment.

### DESCRIPTION OF THE EMBODIMENTS

### [First Embodiment]

With reference to the drawings, a first embodiment will be described.

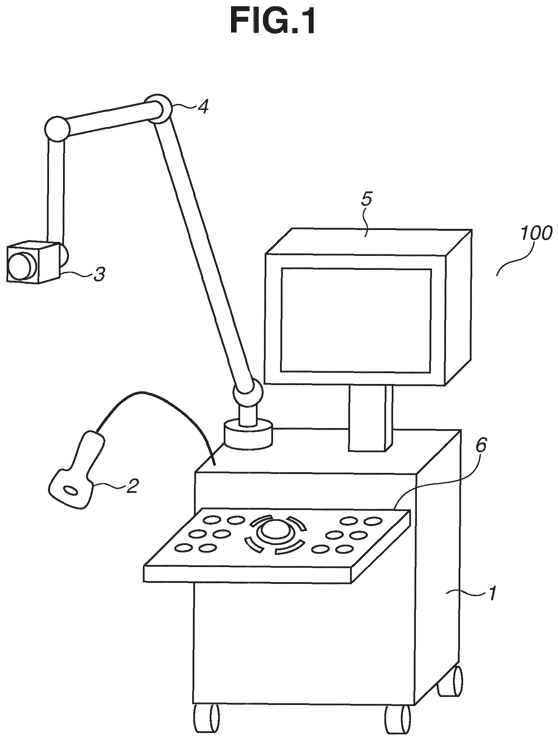

Fig. 1 is a diagram illustrating the entirety of an ultrasound diagnosis apparatus 100 as an example of an inspection system according to the present embodiment. An information processing apparatus according to the present invention is applicable to any electronic device capable of processing a captured image. Examples of the electronic device may include a mobile phone, a tablet terminal, a personal computer, a watch-type information terminal, and an eyeglass-type information terminal.

The ultrasound diagnosis apparatus 100 includes an ultrasound diagnosis apparatus main body 1, an ultrasound probe 2, a camera 3, an arm 4, a display 5, and a control panel 6. The ultrasound diagnosis apparatus main body 1 is configured such that a computer as the information processing apparatus including various control units, a power supply, and a communication interface (I/F) is built into a housing.

The ultrasound probe 2 as an example of an inspection device according to the present embodiment is an ultrasound probe that transmits and receives an ultrasound wave in the state where the surface of the end portion of the ultrasound probe is in contact with the surface of a human subject. The ultrasound probe 2 has a plurality of piezoelectric vibrators built in, which is arranged one-dimensionally (in a line) on the surface of the end portion of the ultrasound probe 2. The ultrasound probe 2 scans a scanning area while transmitting an ultrasound wave to the inside of the human subject using the piezoelectric vibrators, and receives as an echo signal a reflected wave from the human subject. Examples of the scanning technique include B-mode scanning, Doppler mode scanning and various other scanning techniques, and any of the techniques may be used.

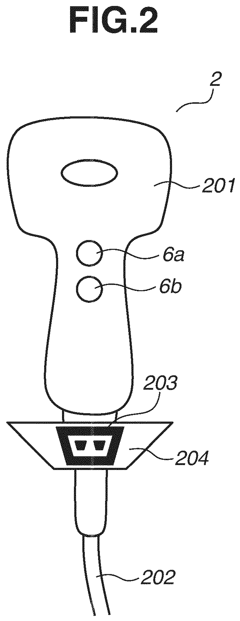

Fig. 2 illustrates an external view of the ultrasound probe 2. The ultrasound probe 2 includes an ultrasound probe main body 201, a connector 202, a marker 203, a marker attachment 204, a freeze button 6a, and a finalize button 6b.

The camera 3 is installed at the end of the arm 4 installed in the ultrasound diagnosis apparatus main body 1 and can be used to capture the state surrounding the ultrasound diagnosis apparatus 100. In the present embodiment, the camera 3 is mainly used to, when the human subject (a subject) is inspected using the ultrasound probe 2, acquire an external appearance image for identifying an inspection part. Specifically, when the human subject is inspected using the ultrasound probe 2, the camera 3 captures an external appearance image including an inspection part of the human subject and the ultrasound probe 2.

The camera 3 includes the components of a general camera, such as an imaging optical system, an image sensor, a central processing unit (CPU), an image processing circuit, a read-only memory (ROM), a random-access memory (RAM), and at least one communication I/F. The camera 3 captures an image as follows. The imaging optical system including an optical element such as a lens forms a light beam from an object into an image on the image sensor including a charge-coupled device (CCD) or a complementary metal-oxide-semiconductor (CMOS) sensor. The imaging optical system includes a lens group, and the camera 3 also includes a lens driving control unit that drives the lens group in the optical axis direction to control the zoom and the focus. An analog-to-digital (A/D) converter converts an electric signal output from the image sensor into digital image data. The image processing circuit performs various types of image processing on the digital image data and outputs the resulting digital image data to an external apparatus. At least a part of the image processing performed by the image processing circuit may be performed by a processing unit of the external apparatus as follows. The image processing circuit outputs the digital image data to the external apparatus via the communication I/F, and then, the processing unit of the external apparatus processes the digital image data.

In the present embodiment, the camera 3 mainly uses an image sensor that receives light in the visible range and captures an image. An example of the camera 3, however, is not limited thereto. Alternatively, the camera 3 may be a camera that captures an image by receiving light in the infrared range, or may be a camera that captures an image by receiving light in a plurality of wavelength ranges, such as visible light and infrared light. Yet alternatively, the camera 3 may be a stereo camera capable of measuring a distance in addition to capturing an external appearance image, or may be a camera including a time-of-flight (TOF) sensor to measure a distance. Hereinafter, an image captured by the camera 3 will be referred to as a "camera image".

The arm 4 is installed in the ultrasound diagnosis apparatus main body 1 and used to place the camera 3 at and in the position and orientation where the camera 3 can capture an external appearance image including an inspection part of the human subject and the ultrasound probe 2. In the present embodiment, the arm 4 is an arm with a serial link mechanism having five joints. The joint at the end of the arm 4 to which the camera 3 is connected is a ball joint allowing the orientation of the camera 3 to be easily set.

The display 5 includes a display device such as a liquid crystal display (LCD) and displays on the display device an image input from the ultrasound diagnosis apparatus main body 1, a menu screen, and a graphical user interface (GUI). The display 5 displays on the display device an image stored in a memory 8 and an image recorded in a non-volatile memory 9. The display 5 is an apparatus that displays an ultrasound image, a camera image, a body mark image, a probe mark image, and the identification result of a part based on control by a CPU 7. The body mark image is an image simply representing the shape of a body and is generally used in an ultrasound diagnosis apparatus. The probe mark is a mark displayed in a superimposed manner on the body mark image and is provided for the purpose of instantly identifying at which angle the ultrasound probe 2 is in contact with the tangent plane of the body.

The control panel 6 includes a keyboard, a trackball, a switch, a dial, and a touch panel. Using these operation members, the control panel 6 receives various input operations from an inspector, such as image capturing instructions to capture an image using the ultrasound probe 2 and capture an image using the camera 3, and instructions to display various images, switch images, specify the mode, and make various settings. In the present specification, an inspector means a doctor, a nurse, or any other user or person that is trained/authorised to use the ultrasound diagnosis apparatus 100/inspection system. Received input operation signals are input to the ultrasound diagnosis apparatus main body 1 and reflected on control of the components by the CPU 7. In a case where the control panel 6 includes a touch panel, the control panel 6 may be integrated with the display 5. In this case, by performing a touch or drag operation on a button displayed on the display 5, the inspector can make various settings of the ultrasound diagnosis apparatus main body 1 and perform various operations on the ultrasound diagnosis apparatus main body 1.

If the inspector operates the freeze button 6a in a state where the state where an ultrasound image in the memory 8 is updated by receiving a signal from the ultrasound probe 2, the signal from the ultrasound probe 2 stops, and the update of the ultrasound image in the memory 8 is temporarily stopped. At the same time, a signal from the camera 3 also stops, and the update of a camera image in the memory 8 is temporarily stopped. If the freeze button 6a is operated in the state where the update of the camera image in the memory 8 is stopped, the signal is received from the ultrasound probe 2 again, the update of the ultrasound image in the memory 8 is started, and similarly, the update of the camera image is also started. When a single ultrasound image is determined by pressing the freeze button 6a, the CPU 7 stores the ultrasound image in the non-volatile memory 9 upon operation on the finalize button 6b by the inspector. The freeze button 6a and the finalize button 6b may be included not in the control panel 6, but in the ultrasound probe 2.

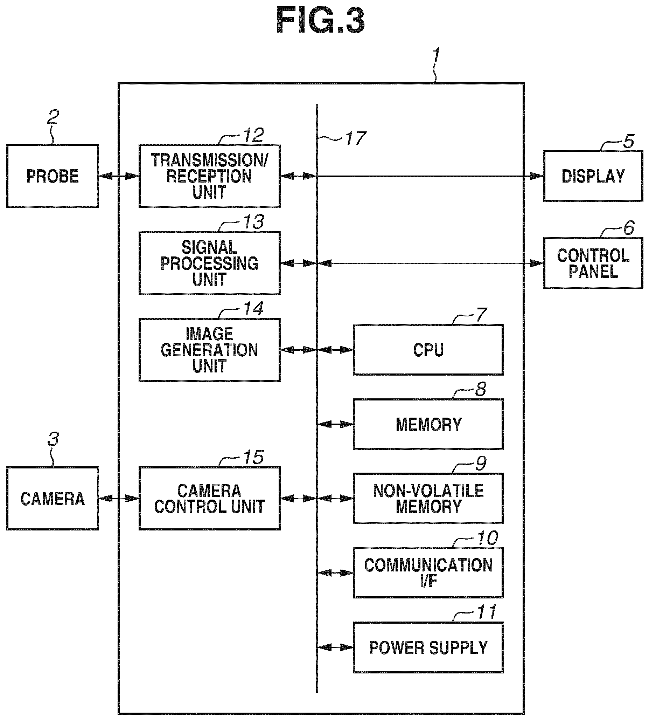

Fig. 3 is a block diagram illustrating the configuration of the ultrasound diagnosis apparatus main body 1. The ultrasound diagnosis apparatus main body 1 includes a transmission/reception unit 12, a signal processing unit 13, an image generation unit 14, a camera control unit 15, the CPU 7, the memory 8, the non-volatile memory 9, a communication I/F 10, and a power supply 11, which are connected to an internal bus 17. The components connected to the internal bus 17 are configured to exchange data with each other via the internal bus 17.

The memory 8 is composed of, for example, a RAM (a volatile memory using a semiconductor device, etc.). For example, according to a program stored in the non-volatile memory 9, the CPU 7 controls the components of the ultrasound diagnosis apparatus main body 1 using the memory 8 as a work memory. The non-volatile memory 9 stores image data, data of the human subject (the subject), and various programs for the operation of the CPU 7. The non-volatile memory 9 is composed of, for example, a hard disk (HD) or a ROM.

The transmission/reception unit 12 includes at least one communication I/F to supply power to the ultrasound probe 2, transmit a control signal, and receive an echo signal. For example, based on a control signal from the CPU 7, the transmission/reception unit 12 supplies to the ultrasound probe 2 a signal for transmitting an ultrasound beam. Further, the transmission/reception unit 12 receives a reflection signal, i.e., an echo signal, from the ultrasound probe 2, performs phasing addition on the received signal, and outputs a signal acquired by the phasing addition to the signal processing unit 13.

The signal processing unit 13 includes a B-mode processing unit (or a BC-mode processing unit), a Doppler mode processing unit, and a color Doppler mode processing unit. The B-mode processing unit visualizes amplitude information regarding a reception signal supplied from the transmission/reception unit 12 by a known process to generate data of a B-mode signal. The Doppler mode processing unit extracts a Doppler shift frequency component from a reception signal supplied from the transmission/reception unit 12 by a known process and further performs a fast Fourier transform (FFT) process, thereby generating data of a Doppler signal of bloodstream information. The color Doppler mode processing unit visualizes bloodstream information based on a reception signal supplied from the transmission/reception unit 12 by a known process, thereby generating data of a color Doppler mode signal. The signal processing unit 13 outputs the generated various types of data to the image generation unit 14.

Based on data supplied from the signal processing unit 13, the image generation unit 14 generates a two-dimensional or three-dimensional ultrasound image regarding a scanning area by a known process. For example, the image generation unit 14 generates volume data regarding the scanning area from the supplied data. The image generation unit 14 generates data of a two-dimensional ultrasound image from the generated volume data by a multi-planar reconstruction (MPR) process or generates data of a three-dimensional ultrasound image from the generated volume data by a volume rendering process. The image generation unit 14 outputs the generated two-dimensional or three-dimensional ultrasound image to the display 5. Examples of the ultrasound image include a B-mode image, a Doppler mode image, a color Doppler mode image, and an M-mode image.

Each of the transmission/reception unit 12, the signal processing unit 13, the image generation unit 14, and the camera control unit 15 in Fig. 3 may be achieved by hardware such as an application-specific integrated circuit (ASIC) or a programmable logic array (PLA). Alternatively, each unit may be achieved by a programmable processor such as a CPU or a microprocessor unit (MPU) executing software. Yet alternatively, each unit may be achieved by the combination of software and hardware.

The camera control unit 15 includes at least one communication I/F to supply power to the camera 3, transmit and receive a control signal, and transmit and receive an image signal. Alternatively, the camera 3 may not receive the supply of power from the ultrasound diagnosis apparatus main body 1, and may include a power supply for driving the camera 3 alone. The camera control unit 15 can control various imaging parameters such as the zoom, the focus, and the aperture value of the camera 3 by transmitting control signals to the camera 3 via the communication I/F. A configuration may be employed in which the camera 3 includes a pan head that allows the camera 3 to automatically perform pan and tilt operations, the camera 3 may be configured to receive a pan/tilt control signal so that the position and orientation of the camera 3 are controlled by pan/tilt driving. Additionally, a driving unit and a driving control unit for electrically controlling the position and orientation of the camera 3 may be included at the end of the arm 4, and the position and orientation of the camera 3 may be controlled based on a control signal from the camera control unit 15 or the CPU 7.

### <Flow of Processing>

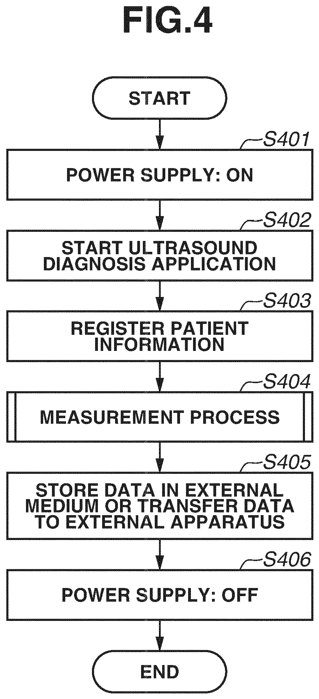

Fig. 4 is a flowchart illustrating a processing flow of the CPU 7 for performing the operation of the entirety of an inspection process by the ultrasound diagnosis apparatus 100. That is, the following steps are executed by the CPU 7 or by components according to an instruction from the CPU 7.

In step S401, according to an operation of the inspector, the CPU 7 turns on the power supply, loads an operating system (OS) stored in the non-volatile memory 9, and starts the OS. Then, in step S402, the CPU 7 automatically starts an ultrasound diagnosis application. At this time, the CPU 7 transmits an image signal of a start screen to the display 5 to cause the display 5 to display the start screen thereon.

After starting the ultrasound diagnosis application, the CPU 7 causes the display screen of the display 5 to transition to a human subject information registration screen after performing an initialization process. In step S403, according to an operation of the inspector on the control panel 6, the CPU 7 receives a registration instruction to register human subject information. The human subject information indicates an inspection part (e.g., mammary gland, heart, artery, abdomen, carotid artery, thyroid gland, and vein) according to the medical condition of the human subject, the human subject identification (ID), the name, the gender, the date of birth, the age, the height, the weight, and whether the human subject is an inpatient or an outpatient. If a start button in the control panel 6 (on the display 5 or the operation panel) is pressed by an operation of the inspector after the human subject information is input, the CPU 7 stores the human subject information in the memory 8 or the non-volatile memory 9. Then, the CPU 7 causes the display screen of the display 5 to transition to a measurement screen based on the ultrasound diagnosis application.

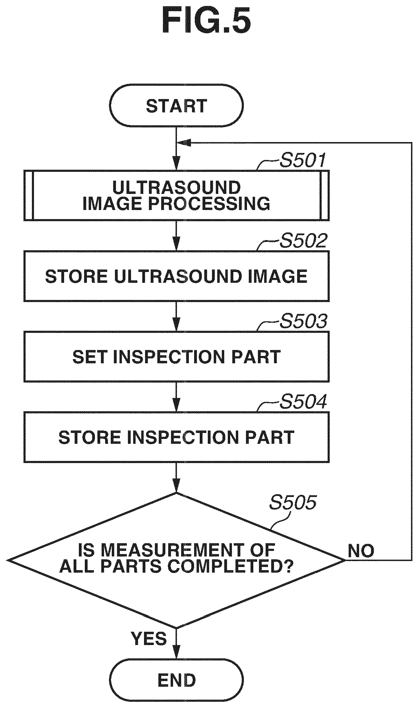

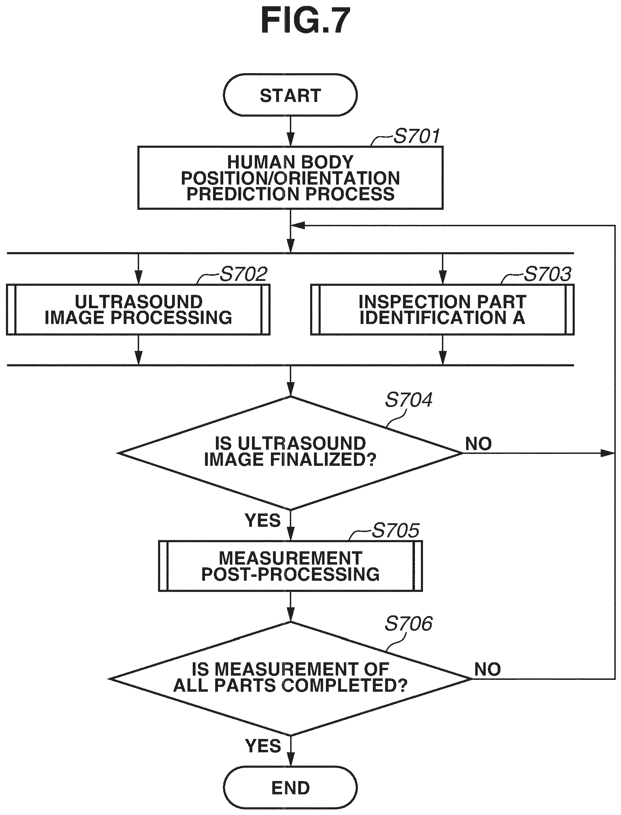

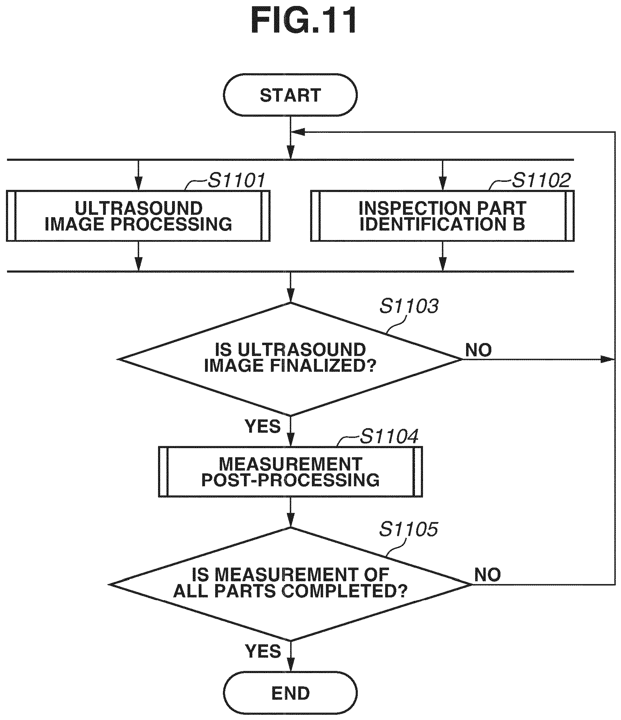

In step S403, the CPU 7 also receives a setting for manually setting an inspection part or a setting for automatically setting an inspection part. The flow of processing to be performed in a case where the setting for manually setting an inspection part is made will be described below with reference to Fig. 5. The flows of processing to be performed in a case where the setting for automatically setting an inspection part (a first variation and a second variation) will be described below with reference to Figs. 7 and 11.

After the display screen transitions to the measurement screen of the ultrasound diagnosis application, then in step S404, a measurement process in an ultrasound diagnosis based on an operation of the inspector is performed. The details of the measurement process will be described below.

If the inspection of all the parts is completed, then in step S405, the CPU 7 stores inspection data obtained by the inspection in the non-volatile memory 9 or an external medium (not illustrated) or transfers the inspection data to an external apparatus (an external server) via the communication I/F 10.

If all the processing is completed, and the operation of turning off the power supply is performed by the inspector, then in step S406, the CPU 7 performs an end process for ending the ultrasonic inspection application and the OS. Thus, a series of processes is ended.

Fig. 5 is a flowchart illustrating the flow of the measurement process in step S404 in Fig. 4 in a case where the inspector makes the setting for manually setting an inspection part. The following steps are executed by the CPU 7 or by components according to an instruction from the CPU 7.

In step S501, the CPU 7 performs signal processing and image processing on an echo signal received from the ultrasound probe 2, thereby generating an ultrasound image. Then, the CPU 7 displays the ultrasound image on the display 5. The details of the ultrasound image processing in step S501 will be described below.

The inspector confirms the ultrasound image displayed on the display 5. Then, in the state where a desired ultrasound image can be obtained, the CPU 7 finalizes the ultrasound image according to an operation of the inspector. In step S502, the CPU 7 stores the ultrasound image in the memory 8.

In step S503, to record information indicating where the inspected part is, an inspection part is set according to an operation of the inspector using a body mark or a probe mark. Further, an annotation such as a comment or an arrow may be input to the display 5 according to an operation of the inspector.

If the finalize button 6b is pressed by an operation of the inspector, then in step S504, information regarding the inspection part is stored in the memory 8.

If the measurement process for measuring a certain inspection part is completed, then in step S505, the CPU 7 determines whether all the inspection parts determined in advance according to the inspection content are measured. If any part has remained uninspected (NO in step S505), the processing returns to the measurement process in step S501. Information regarding the inspection content is selected and set according to an operation of the inspector from pieces of information classified according to inspection parts or conditions and recorded in the non-volatile memory 9 in advance. If it is determined that the measurement process for measuring all the inspection parts is completed (YES in step S505), the process of step S404 is ended.

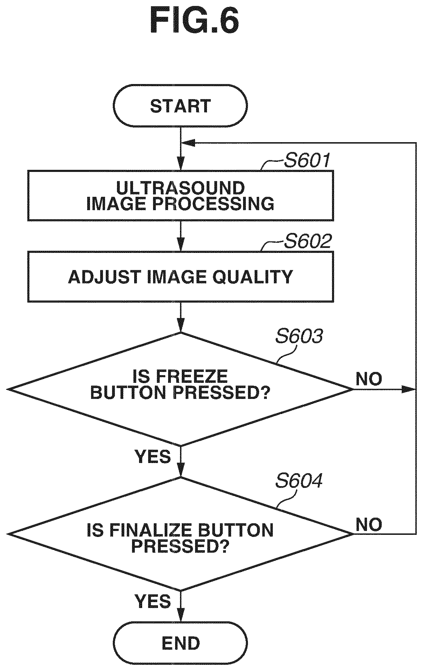

Fig. 6 is a flowchart illustrating the detailed flow of the ultrasound image processing in step S501. The following steps are executed by the CPU 7 or by the signal processing unit 13 and the image generation unit 14 according to an instruction from the CPU 7.

As described above, the ultrasound probe 2 is an example of the inspection device. The ultrasound probe 2 scans a scanning area while transmitting an ultrasound wave to the inside of the human subject using the piezoelectric vibrators, and receives as an echo signal (an ultrasound signal) a reflected wave from the human subject. In the present embodiment, the ultrasound probe 2 can be operated by the inspector holding the ultrasound probe 2 in the hand. In step S601, the signal processing unit 13 and the image generation unit 14 perform signal processing and image processing on an ultrasound signal transmitted from the ultrasound probe 2, thereby generating an ultrasound image. Then, the CPU 7 displays the ultrasound image on the display 5. To obtain a desired image, the inspector can further correct the ultrasound image by adjusting various processing parameters using the control panel 6 while confirming the ultrasound image displayed on the display 5. That is, in step S602, various parameters (e.g., the mode, the gain, the focus, and the echo level) are changed according to operation signals received by the control panel 6, and an ultrasound image after the changes is regenerated and displayed on the display 5.

In step S603, the CPU 7 determines whether the freeze button 6a, which is provided in the ultrasound probe 2, is pressed. If the freeze button 6a is not pressed (NO in step S603), steps S601 and S602 are repeated. If the freeze button 6a is pressed (YES in step S603), the CPU 7 displays on the display 5 the ultrasound image captured and generated at this time on the assumption that a desired ultrasound image is acquired. Then, the processing flow of the ultrasound image processing ends.

### (Variation 1)

Fig. 7 is a flowchart illustrating the detailed flow of the measurement process in step S404 in Fig. 4 in a case where the inspector makes the setting for automatically setting an inspection part. The following steps are executed by the CPU 7 or by components according to an instruction from the CPU 7.

In the first variation, the setting of an inspection part made according to an operation of the inspector in step S503 in Fig. 5 is automated.

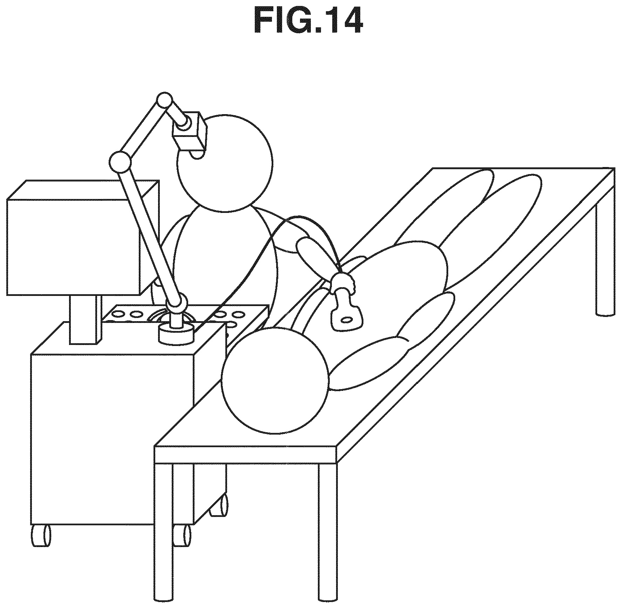

In step S701, the CPU 7 causes the camera control unit 15 to start and control the camera 3 to capture an image including the human subject. Fig. 14 illustrates an image diagram of a measurement using the inspection system according to the present embodiment. At this time, the inspector moves the arm 4 to place the camera 3 at and in appropriate position and orientation where a portion of the human subject is included in the angle of view of the camera 3. The inspector then captures an image using the camera 3 by an operation using an operation member such as a shutter button disposed in advance in the control panel 6 or the camera 3. The present invention is not limited thereto. Alternatively, at least one of steps for controlling the position and orientation of the camera 3 and controlling the capturing of an image may be automated. That is, in an embodiment in which the camera 3 with a pan head having a pan/tilt mechanism is attached to the end of the arm 4, first, the camera control unit 15 controls the driving of the camera 3 so that the position and orientation of the camera 3 are appropriate position and orientation. Specifically, the human subject is detected by image analysis from a captured image obtained at and in the current position and orientation of the camera 3, and the position and orientation of the camera 3 are controlled by pan/tilt control so that a portion of the human subject is included in the angle of view of the camera 3. If a portion of the human subject cannot be detected from the captured image obtained at and in the current position and orientation of the camera 3, the movement of the camera 3 and the capturing of an image are repeated a predetermined number of times by panning and tilting the camera 3 to different angles of view until a portion of the human subject is detected. After the camera 3 is controlled to be at and in appropriate position and orientation, the CPU 7 causes the camera 3 to capture an external appearance image including a portion of the human subject and acquires the external appearance image. Further, if captured images at a plurality of angles of view (a plurality of points of view and a plurality of positions and orientations) are required for a prediction process for predicting the position and orientation of the human subject, the driving of the camera 3 may be controlled so that the camera 3 is at and in a plurality of positions and orientations, and then, the camera 3 may be caused to capture an image a plurality of times.

Based on the external appearance image acquired from the camera 3, the CPU 7 predicts the position and orientation of the human subject lying on a bed and displays the prediction result on the display 5. If the prediction result of the position and orientation of the human subject is finalized according to an operation of the inspector on the control panel 6, the CPU 7 stores the position and orientation of the human subject at this time in the memory 8. Descriptions will be given below of the details of the prediction process for predicting the position and orientation of the human subject included within the angle of view, a prediction process for predicting the position and orientation of the ultrasound probe 2, and an identification process for identifying an inspection part based on both prediction results.

Then, the flow of ultrasound image processing in step S702 and the flow of inspection part identification A in step S703 are processed in parallel.

Step S702 is a flow equivalent to the flow of the ultrasound image processing in step S501 described above with reference to Fig. 6.

In step S703, the CPU 7 automatically predicts an inspection part using the external appearance image acquired from the camera 3 and displays the prediction result on the display 5. The details will be described below.

If the freeze button 6a in the ultrasound probe 2 is pressed by an operation of the inspector, the update of the ultrasound image on the display 5 based on the ultrasound signal from the ultrasound probe 2 is stopped and finalized. Simultaneously with or subsequently to the finalization of the ultrasound image, the CPU 7 displays on the display 5 the prediction result of the inspection part when the freeze button 6a is pressed. Then, the processes in steps S702 and S703 are ended.

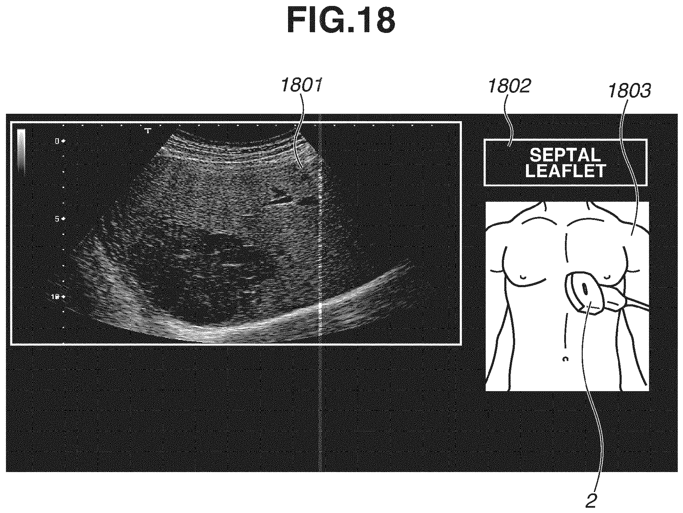

In step S704, the CPU 7 receives a finalization instruction to finalize the ultrasound image in the state where the ultrasound image, the external appearance image, and inspection part identification (prediction) information that are obtained in steps S701 to S703 are displayed on the display 5 as illustrated in Fig. 18. An ultrasound image 1801 and a display external appearance image 1803 are sequentially updated in the respective cycles and displayed on the display 5. As inspection part identification information 1802, part information at the current time that is identified (or is predicted but has not yet been identified by an instruction from the inspector) in step S703 is displayed on the display 5. The CPU 7 may display the display external appearance image 1803 by clipping, rotating, or resizing a portion of the external appearance image from the camera 3 based on general inspection part information received in advance in step S403 or the identified part information. If the ultrasound image is a desired ultrasound image, the finalize button 6b is pressed by an operation of the inspector (YES in step S704), and the processing proceeds to step S705. In step S705, the CPU 7 performs post-processing such as recording and displaying. If the ultrasound image is not a desired ultrasound image, the inspector does not press the finalize button 6b, or performs another predetermined operation (NO in step S704), and the processing returns to the parallel processing in steps S702 and S703.

In step S706, the CPU 7 determines whether all the inspection parts determined in advance according to the inspection content are measured. If there is any part that has remained uninspected (NO in step S706), the processing returns to the parallel processing in steps S702 and S703. In the present embodiment, the inspection content for inspecting a plurality of inspection parts is stored in advance in the non-volatile memory 9, and it is determined whether all the inspection parts determined in advance according to the inspection content are measured in step S706. Alternatively, a form may be employed in which if the capturing and the storing of a single inspection part are completed, the above determination is not made, and the flow is ended.

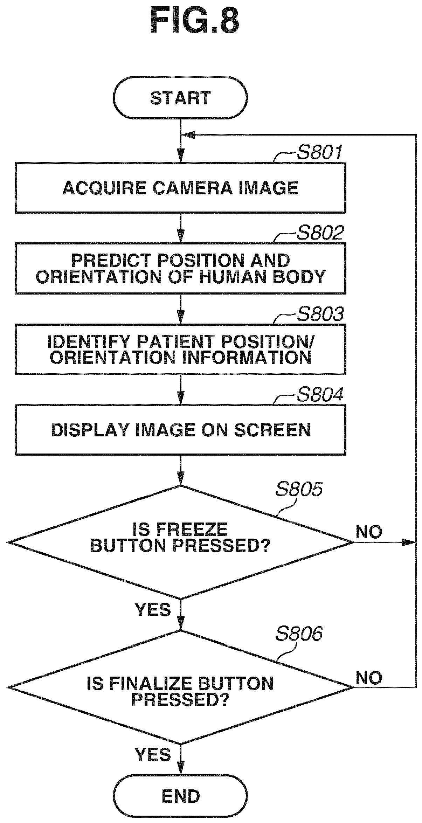

Fig. 8 is a flowchart illustrating the detailed flow of the prediction process for predicting the position and orientation of the human body in step S701 in Fig. 7. The following steps are executed by the CPU 7 or by components according to an instruction from the CPU 7.

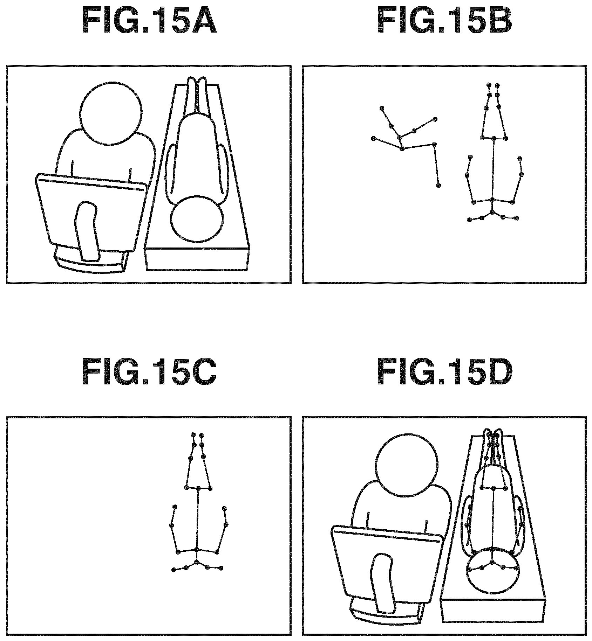

In step S801, the CPU 7 causes the camera control unit 15 to control the camera 3 to capture the human subject lying on a bed as illustrated in Fig. 15A. The camera 3 sequentially captures images at a predetermined frame rate, and the CPU 7 receives external appearance images via the communication I/F of the camera control unit 15 and sequentially displays the external appearance images on the display 5.

While confirming the external appearance image displayed on the display 5, the inspector adjusts the position of the arm 4 so that an inspection part of interest which is at least a portion of the human subject is included within the angle of view of the camera 3. The display 5 may display a line for guiding the inspector as to which position in the displayed external appearance image the inspection part of the human subject should be located at by the inspector adjusting the arrangement of the camera 3. At this time, the line for guiding the inspector (i.e., GUI data to be superimposed on the display image) is stored in advance in the non-volatile memory 9 in association with information regarding the inspection part.

In step S802, based on an image from the camera 3 acquired as the external appearance image, the CPU 7 predicts the position and orientation of the human body by an image analysis process. In the present embodiment, as position/orientation information regarding the human body, skeleton information including the position coordinates of feature points such as joints is output. The joints are the nose, the neck, the right shoulder, the right elbow, the right wrist, the left shoulder, the left elbow, the left wrist, the center of the hip, the right portion of the hip, the right knee, the right ankle, the left portion of the hip, the left knee, the left ankle, the right eye, the left eye, the right ear, the left ear, the left thumb, the left little finger, the left heel, the right thumb, the right little finger, and the right heel. As a method for obtaining the skeleton information from the image, a learner trained using a machine learning (deep learning) method is used. In the present embodiment, a learning model (a learner) trained in advance using a set of a plurality of images of training data including a human body as a subject and correct answer information of skeleton information (the probability distribution of each joint) in each image of the training data is used. That is, a learning model is trained in advance using a set of a plurality of images of training data including a subject and correct answer information. In other words, the human bodies as the subject that are used for the training are similar to a subject to be inspected. In this method, information obtained from a camera (including a stereo camera, an infrared camera, and a TOF camera) may be only a luminance image, only a depth image, or both a luminance image and a depth image. In any case, the skeleton information can be acquired based on two-dimensional (2D) coordinates or three-dimensional (3D) coordinates. As such a learner, for example, OpenPose (registered trademark) of Carnegie Mellon University is known. In the present embodiment, the position/orientation information (the skeleton information) predicted by the learner trained using machine learning is stored in the form of an array or a list in a memory. Specifically, information indicating the distribution of the probabilities of the presence of each of a plurality of parts such as the above joints in the image is output as the predicted position/orientation information. If the distribution of the reliabilities (the probability distribution) of the presence of a part n (n is an integer) on the image is Rn(x,y), an output R as the skeleton information is represented as R = {Rn(x,y)|n = 1, 2, ..., N, N is an integer}. Alternatively, Rn(x,y) may not be the distribution of the reliabilities in the entire area of the image, and may be the distribution of the reliabilities in only an area having a reliability greater than a threshold. Yet alternatively, only the peak value of the reliabilities may be stored as Rn(x,y) in association with the coordinates of the peak value (e.g., a part 3: the right shoulder, the reliability: 0.5, the coordinates: (x,y) = (122,76)).

Fig. 15B illustrates an example of an image obtained by, in the output R, extracting the position of the peak value of the reliabilities of each part (i.e., the position where the highest probability of the presence of each part is detected) and visualizing the skeleton information based on information regarding the extracted position.

In the present embodiment, as preprocessing for obtaining the skeleton information from the image using the trained learner, the correction of the image quality such as noise removal, distortion correction, color conversion, luminance adjustment, or color gradation correction, and the rotation or the flipping of the image are performed. Parameters for the correction are stored as a table in the non-volatile memory 9 according to the model of the camera 3 that captures an image, and the imaging conditions when an image is captured. The correction process is performed on the input external appearance image using these correction parameters, and the external appearance image is brought close to the imaging conditions of the images in the data set used in the training, thereby performing an inference with higher accuracy. For example, a case is possible where in an image captured in a dark room, high sensitivity noise occurs when the image is corrected to be bright, and the tendency of the image differs from that of the data set used in the training. In such a case, the process of removing high sensitivity noise can be performed on the input external appearance image. Similarly, in a case where the lens of the camera 3 has a wide angle of view, and a peripheral portion of the input external appearance image is greatly distorted, the distortion can be corrected. In a case where the head is on the upper side in all the images included in the data set, the images can be input after rotating or flipping the images so that the head is on the upper side. In a case where the learner is trained using an image obtained by converting an image by some process, the input image can also be similarly converted and then input to the learner.

As illustrated in Fig. 15B, in step S802, skeleton information regarding the inspector or a human being around the human subject may also be acquired together. Thus, in step S803, the CPU 7 identifies skeleton information regarding the human subject from an image of the skeleton information obtained in step S802.

As an identification method for identifying the skeleton information regarding the human subject, for example, the following methods are possible. One of the methods is a method combined with a face authentication process. The CPU 7 authenticates the face of the inspector registered in advance in the non-volatile memory 9 from the external appearance image. Based on the distance relationships between the location where the face of the inspector is authenticated and detected, and portions detected as parts of a face (the eyes, the nose, and the ears) in the skeleton information detected in step S802, the skeleton information regarding the inspector and the skeleton information regarding the human subject are identified.

As another method, the ultrasound diagnosis apparatus 100 may be identified, and the inspector and the human subject may be distinguished based on the planar (XY directions in the image) or three-dimensional (XYZ directions) distance relationships between the ultrasound diagnosis apparatus 100 and the inspector and the human subject. As yet another method, the types of orientations are identified based on the relationships between the positions of joint points in the skeleton information, and the inspector and the human subject are distinguished. As yet another method, a procedure is defined so that the human subject appears in a determined area when the angle of view of the camera 3 is adjusted, and the human subject is identified based on the position of the skeleton information.

In the present embodiment, the inspector and the human subject are identified by executing at least one of the above distinction techniques. Then, position/orientation information regarding the identified human subject is visualized as illustrated in Fig. 15C.

In step S804, the CPU 7 displays on the display 5 an image as illustrated in Fig. 15D obtained by superimposing the thus predicted position/orientation information regarding the human subject on the display image from the camera 3.

As the image displayed on the display 5 by the CPU 7, the image acquired by the camera 3 may not be directly used, but may be displayed by replacing the image with an avatar or an animation or converting the image into a 3D model by known image processing in the interest of privacy.

In step S805, if the freeze button 6a is pressed according to an operation of the inspector (YES in step S805), the update of the prediction result of the skeleton information (the position/orientation information) regarding the human subject based on the external appearance image sequentially displayed on the display 5 is ended, and the processing proceeds to step S806. If the freeze button 6a is not pressed (NO in step S805), the processing returns to step S801. In step S801, the CPU 7 continues to predict the position/orientation information.

After the freeze button 6a is pressed in step S805, then in step S806, if the inspector confirms that there is no problem with the prediction result of the position/orientation information displayed on the display 5, and operates the finalize button 6b (YES in step S806), the prediction process for predicting the position and orientation of the human body is ended. If the prediction result of the position and orientation is not a desired result in step S806 (NO in step S806), the processing returns to step S801. In step S801, the CPU 7 repeatedly predicts the position and orientation.

The reason for waiting for the inspector's confirmation in step S806 is to deal with a case where the orientation of the human subject is not a desired orientation, a case where the camera angle is wrong, or a case where the detection result of the position and orientation deviates significantly from what the human eyes see.

In another embodiment, steps S805 and S806 may be omitted (ignored). That is, until the inspector determines that the identification process for identifying an inspection part at the subsequent stage is appropriate, and the inspector performs a finalization operation, the position/orientation information regarding the human body may continue to be updated at predetermined time intervals.

In the present embodiment, as the method for predicting the position and orientation of the human body, the skeleton information (joint information) is used. Alternatively, the 3D shape of a human being may be predicted based on meshed information. Such a technique can also be achieved using machine learning (a machine learner). That is, a learner trained in advance using a set of a plurality of images of training data including a subject and correct answer (label) information of meshed information in each image of the training data may be used.

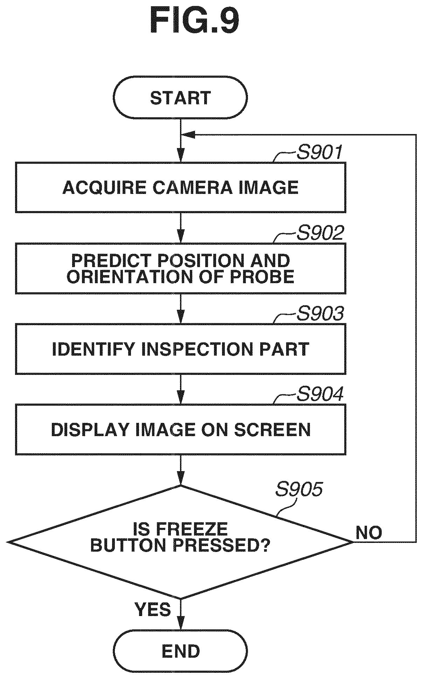

Fig. 9 is a flowchart illustrating the detailed operation of the inspection part identification A in step S703 in Fig. 7. The following steps are executed by the CPU 7 or by components according to an instruction from the CPU 7.

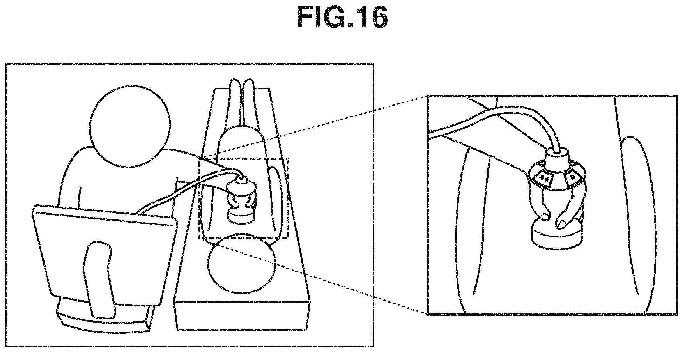

In step S901, the CPU 7 acquires an external appearance image (image data) including the ultrasound probe 2 from the camera 3 via the communication I/F of the camera control unit 15. Since the angle of view of the camera 3 is adjusted to include the human subject in the previous steps, an image may be captured without changing the angle of view. However, at least one of pan control, tilt control, and zoom control may be performed to obtain an angle of view that allows the detection of the ultrasound probe 2 easier. Fig. 16 is an image diagram illustrating a change in the angle of view of the camera 3 in a case where the angle of view is adjusted from an external appearance image mainly including the human subject to an external appearance image mainly including the ultrasound probe 2, and captured images.

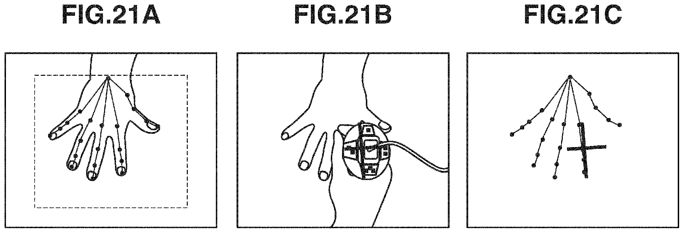

In step S902, the CPU 7 analyzes the acquired external appearance image to obtain the position and orientation of the ultrasound probe 2. Specifically, the CPU 7 detects a plurality of augmented reality (AR) markers (a plurality of corners is provided in each marker) provided in the ultrasound probe 2 from the external appearance image by an image recognition process, including a filter process, a binarization process, a determination process and a shape recognition process, for edge detection. To perform image recognition with higher accuracy, the CPU 7 may further perform image processing such as a sharpness process, a gain process, and a noise reduction process on the external appearance image acquired from the camera 3. The CPU 7 detects each of the AR markers provided in the ultrasound probe 2 and calculates the position and orientation of the ultrasound probe 2 based on the positional relationship between the plurality of corners present in the detected AR marker, and the sizes and the distorted states of figures formed by the corners. Since the ultrasound probe 2 is a rigid body, the relationships between the AR markers and the inspection surface of the ultrasound probe 2 can be obtained by calculation. A plurality of (preferably, three or more) AR markers can be placed on the marker attachment 204 as illustrated in Figs. 16 and 21B. The AR markers are placed at predetermined intervals around the ultrasound probe 2 so that at least one of the AR markers can be captured and acquired by the camera 3 regardless of the direction of the ultrasound probe 2 or the arrangement of the connector 202. In the present embodiment, the CPU 7 outputs the following as an output regarding the position and orientation of the ultrasound probe 2. That is, the CPU 7 outputs position/orientation information including position information (image coordinate information (x,y)) in the external appearance image, position information (x,y,z) in a three-dimensional space based on the camera 3, and vector information (a direction vector d = (x,y,z)) indicating the direction of the probe 2. The position information (x,y) is used in the process of identifying an inspection part in step S903. The position/orientation information (x,y,z) or the direction vector d is used to display the position and orientation of the probe 2 relative to the inspection part in a body mark on a screen displayed on the display 5 in step S904, so that the position and orientation can be visually confirmed. The present invention, however, is not limited to this. Alternatively, the position/orientation information may be used in the identification process for identifying an inspection part. Yet alternatively, when the display process is performed, only position information may be simply displayed.

Not only an AR marker but also anything such as an LED or a retroreflective mark can be used as a reference (an indicator) for obtaining the position and orientation of the ultrasound probe 2, so long as the position and orientation of the ultrasound probe 2 can be obtained by calculation.

As described above, generally, the ultrasound probe 2 is a rigid body, and the position and orientation of the ultrasound probe 2 are limited. Thus, the position and orientation of the ultrasound probe 2 are detected by a rule-based process, i.e., image recognition using a predetermined pattern such as an AR marker. This allows high detection accuracy, and it is possible to obtain the position and orientation at a lower processing cost and a higher speed than a technique using a learner trained using machine learning.

In step S903, the CPU 7 identifies an inspection part based on the relationship between the prediction result (R) of the position and orientation of the human subject previously obtained and stored in the memory 8 in step S701, and the position information (x,y) regarding the ultrasound probe 2 obtained in step S902. In the present embodiment, a plurality of candidates for an inspection part are output as identification results in descending order of the evaluation values of parts as the inspection part in the pieces of skeleton information.

As an identification method for identifying an inspection part in a case where the skeleton information R is acquired as information regarding the position and orientation of the human subject, the following method is possible.

For example, the coordinates of the position of the peak value of the reliabilities in the reliability distribution Rn(x,y) of each part, i.e., the coordinates of the position of the highest reliability of each part (simply referred to as "the coordinates of each part"), are extracted. Then, an inspection part is identified based on the distance (the Euclidean distance or the Mahalanobis distance) between the coordinates of each part and the position information regarding the ultrasound probe 2. That is, the evaluation values are calculated so that the smaller the distance between the coordinates of each part and the ultrasound probe 2 is, the greater the evaluation value of the part is. Further, the evaluation values of the parts are calculated so that the greater the reliability corresponding to the coordinates of each part is, the greater the weight of the part is. Then, parts are extracted as candidates for an inspection part in descending order of the evaluation values of the plurality of parts. When the evaluation values are calculated, the evaluation values may be obtained with reference to only either one of the distance from the position of the ultrasound probe 2 and the reliability distribution of each part.

Alternatively, the evaluation values may be calculated in each area in the image using the reliability distribution Rn(x,y) of each part. That is, the evaluation value distribution obtained by Rn(x,y) × (the weight based on the distance from the ultrasound probe 2) may be calculated in each part, and the position of the highest evaluation value and a part corresponding to this position may be identified as an inspection part.

As another technique, the evaluation values may be calculated based on the distances between straight lines connecting the positions of the above parts and the position information (x,y) regarding the ultrasound probe 2, and the reliability distribution of the parts at the pairs of points corresponding to the straight lines, and an inspection part may be identified.

As another technique, the evaluation values may be calculated based on the distances between coordinates obtained by dividing the distances between the positions of a plurality of parts in certain proportions and the position information (x,y) regarding the ultrasound probe 2, and the reliability distribution of the parts at the pairs of points in the divided portions, and an inspection part may be identified.

As another technique, the evaluation values may be calculated by creating a 2D or 3D closed area from points obtained in certain proportions based on the relationships between a plurality of joints, and determining whether the position information (x,y) regarding the ultrasound probe 2 is inside or outside the closed area, and an inspection part may be identified.

Alternatively, the evaluation values may be calculated by combining some or all of the above plurality of techniques.

In step S903, due to the movement of the human subject after step S701, the position and orientation of the human subject stored in the memory 8 and the current position and orientation of the human subject may be different from each other. The inspection part identified using the different positions and orientations may be an incorrect result. Accordingly, it may be determined whether the human subject is moving. If the movement of the human subject is detected, the processing may return to step S701. In step S701, the position and orientation of the human body may be predicted again.

As a method for determining whether the human subject is moving, for example, a method using a subtraction image or a method using an optical flow is used.

In a case where a subtraction image is used, for example, a mask process is performed on the hand of the inspector and a portion of the probe 2, and the remaining portion are examined regarding whether the luminance value or the hue have changed by an amount greater than or equal to a threshold between the images acquired in steps S801 and S901. At this time, the change may be detected by a statistical process.

In a case where an optical flow is used, for example, the human body in the camera image acquired in step S801 when the position and orientation of the human body are predicted is registered as a pattern, and template matching is performed on the camera image in step S901 to detect the movement. Alternatively, the camera image acquired in step S801 is temporarily stored in the memory 8, and the amount of movement of a feature point obtained by scale-invariant feature transform (SIFT) or Accelerated-KAZE (AKAZE) is calculated between the camera image acquired in step S801 and the image acquired in step S901 to detect he movement of the human body in the images.

Alternatively, a known tracking technique such as a Kernelized Correlation Filter (KCF) tracker may be used.

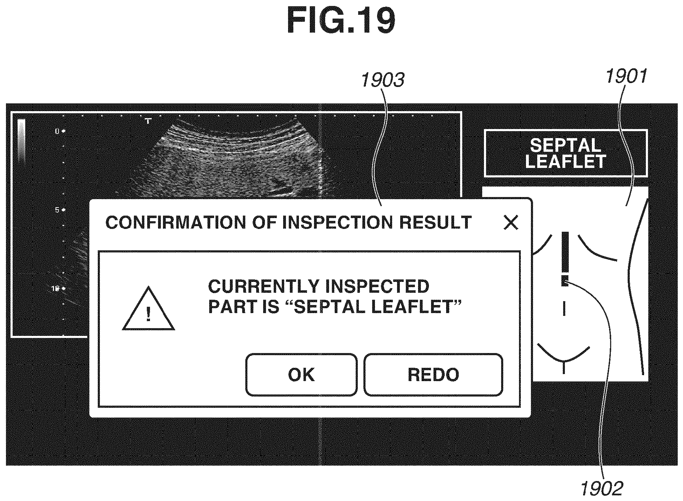

In step S904, the CPU 7 displays on the display 5 the inspection part identified in step S903. In step S905, the CPU 7 confirms whether an operation corresponding to selection of an OK button is performed on the control panel 6, or an operation of pressing the freeze button 6a in the ultrasound probe 2 is performed by the inspector. Fig. 19 illustrates an example of a screen displayed on the display 5 to approve the identification result of the inspection part in step S905. This screen is displayed on the display 5 such that on a GUI body mark 1901 corresponding to the body of the human subject, a GUI probe mark 1902 corresponding to the ultrasound probe 2 is superimposed at the position of the corresponding inspection part. At this time, if the name of the inspection part is also identified, the name ("septal leaflet" in Fig. 19) may be displayed with the body mark 1901 and the probe mark 1902. A confirmation window 1903 for confirming the inspection result is displayed on the screen, and OK and redo icon buttons are displayed in the confirmation window 1903. The inspector selects and specifies the OK or redo button using the control panel 6 or presses the freeze button 6a in the ultrasound probe 2, so as to finalize the inspection part or give an instruction to identify an inspection part again. If an instruction to select the OK button is given, or the freeze button 6a is pressed, a series of processes is ended. If an instruction to select the redo button is given, or the freeze button 6a is not pressed, the processes of step S901 and the subsequent steps for identifying an inspection part are repeated. In step S905, while the window illustrated in Fig. 19 for prompting the inspector to confirm the inspection result is displayed, the process of identifying an inspection part may be interrupted. Alternatively, the finalization process in step S905 may be omitted, and the finalization process for finalizing the inspection part may be performed at any timing in steps S701 to S704.

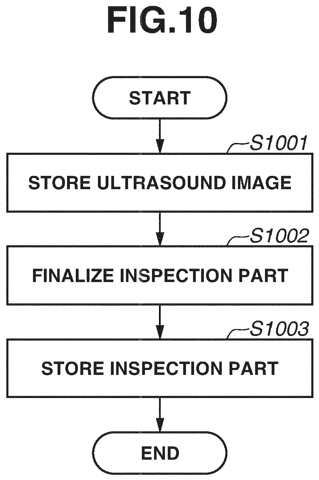

Fig. 10 is a flowchart illustrating the operation after the measurement process in step S705 in Fig. 7. The following steps are executed by the CPU 7 or by components according to an instruction from the CPU 7.

In step S1001, the CPU 7 stores data of the ultrasound image finalized in step S704 in the non-volatile memory 9 or an external medium or transfers the data to outside, and also displays the ultrasound image finalized in step S704 on the display 5.

In step S1002, the inspection part identified by the operation of the inspection part identification A in step S703 is finalized according to an operation of the inspector. At this time, the ultrasound image finalized in step S704 and the body mark and the probe mark displayed in step S904 are displayed simultaneously or switchably on the display 5. The inspector confirms the inspection part and the position/orientation information regarding the probe 2 displayed on the display 5 again. If the inspection part is correct, the inspector presses a predetermined operation member of the control panel 6 or the finalize button 6b in the ultrasound probe 2. If the inspection part is incorrect, on the other hand, the second and third candidates for a part are displayed on the display 5 by an operation of the inspector on the control panel 6, and a corresponding inspection part is selected by an operation of the inspector. Then, the inspection part is finalized by the finalization operation as described above.

In step S1003, the CPU 7 stores information regarding the inspection part finalized in step S1002 and the position/orientation information regarding the probe 2 in the non-volatile memory 9 or an external medium in association with the ultrasound image finalized in step S704 or transfers the information regarding the inspection part and the position/orientation information regarding the probe 2 to outside. Examples of the information regarding the inspection part and the position/orientation information regarding the probe 2 include the name of the inspection part, position/orientation information regarding the body mark, and position/orientation information regarding the probe mark relative to the body mark. The position/orientation information regarding the probe mark may be an angle on a two-dimensional image, or may be three-dimensional orientation information.

### (Variation 2)

Fig. 11 is a flowchart illustrating another form of the measurement process in the ultrasound diagnosis in step S404 in Fig. 4 in a case where the inspector makes the setting for automatically setting an inspection part. In this flow, similarly to the flow in Fig. 7, the setting of an inspection part made according to an operation of the inspector in step S503 in Fig. 5 is automated. The following steps are executed by the CPU 7 or by components according to an instruction from the CPU 7.

The CPU 7 performs the processes of steps S 1101 and S1102 in parallel. Ultrasound image processing in step S1101 is similar to that in step S501 in Fig. 5 (described in detail in Fig. 6). The processing of inspection part identification B in step S1102 will be described below.

In step S1103, if a predetermined operation member of the control panel 6 or the freeze button 6a in the ultrasound probe 2 is pressed by an operation of the inspector (YES in step S1103), the CPU 7 stops the update of the ultrasound image on the display 5. Simultaneously with or subsequently to this, the CPU 7 displays on the display 5 the identification result of an inspection part when the freeze button 6a is pressed, and the processing in steps S1101 and S1102 is ended. Then, the processing proceeds to step S1104. In step S1104, measurement post-processing is performed.

If the ultrasound image is not a desired ultrasound image (NO in step S1103), the processing returns to the parallel processing in steps S1101 and S1102.

When the measurement process for measuring a certain inspection part is completed, then in step S1105, the CPU 7 determines whether all the inspection parts determined in advance according to the inspection content are measured. If there is any part that has remained uninspected (NO in step S1105), the processing returns to the parallel processing in steps S1101 and S1102.

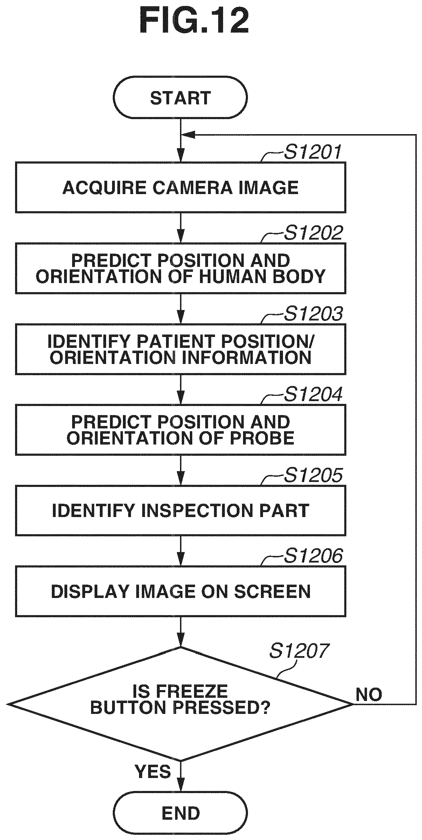

Fig. 12 is a flowchart illustrating the operation of the inspection part identification B in step S1102 in Fig. 11.

The steps of performing basically the same operations as those in the processes illustrated in the flow in Fig. 9 are not described. Step S901 corresponds to step S1201. Step S902 corresponds to step S1204. Step S903 corresponds to step S1205. Step S904 corresponds to step S1206. Step S905 corresponds to step S1207.

The flow in Fig. 12 is different from the flow in Fig. 9 in that in step S1202, a prediction process for predicting the position and orientation of the human body (the human subject), which corresponds to step S701 in Fig. 7, is performed. In Fig. 7, this process is performed outside the flow of the inspection part identification A in step S703.

In the processing in Fig. 12, unlike the processing in Fig. 7, the prediction process for predicting the position and orientation of the human body and the prediction process for predicting the position and orientation of the probe 2 are performed based on a single camera image obtained by the camera 3 capturing an image once, and an inspection part is predicted. Thus, it is possible to reduce the operations of the inspector pressing the freeze button 6a and pressing the finalize button 6b. If, however, an inspection part of the human subject and the ultrasound probe 2 cannot be simultaneously captured, it may be difficult to identify an inspection part. For example, a case is possible where an inspection part is hidden behind the ultrasound probe 2 or the inspector. Further, the update frequency of part identification may be constrained by the time taken for the prediction process for predicting the position and orientation of the human body. In the processing in Fig. 7, in contrast, the camera image for predicting the position and orientation of the human body is different from the camera image for predicting the position and orientation of the probe 2. Thus, it is easy to avoid the above issue.

### (Variation 3)

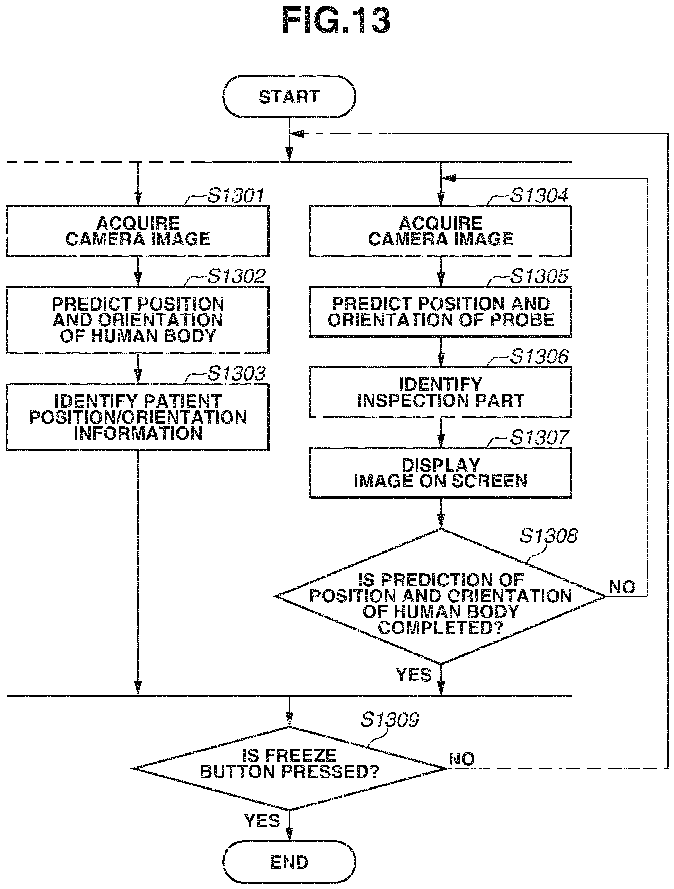

Fig. 13 is a flowchart illustrating the detailed flow of another form of the inspection part identification B in step S1102 in Fig. 11.

The steps of performing basically the same operations as those in the processes illustrated in the flow in Fig. 12 are not described. Step S1201 corresponds to steps S1301 and S1304. Step S1202 corresponds to step S1302. Step S1203 corresponds to step S1303. Step S1204 corresponds to step S1305. Step S1205 corresponds to step S1306. Step S1206 corresponds to step S1307.

Fig. 13 is different from Fig. 12 in that the process of predicting the position and orientation of the human subject in steps S1301 and S1302 and the process of predicting the position and orientation of the ultrasound probe 2 and an inspection part in steps S1304 to S1308 are performed in parallel.

In the present embodiment, the calculation cost of the process of predicting the position and orientation of the human body is higher than the calculation cost of the process of obtaining the position and orientation of the ultrasound probe 2. This is because a rule-based process including a filtering process, a binarization process, and a shape recognition process is performed to calculate the position and orientation of the ultrasound probe 2, and a recognition process using a learner trained using deep learning is used in the prediction process for predicting the position and orientation of the human body (the human subject). Thus, in step S1308 in the present embodiment, until the prediction process for predicting the position and orientation of the human body is completed, only the calculation result of the position and orientation of the ultrasound probe 2 is updated, and an inspection part is identified.

### (Variation 4)

A variation of the identification process for identifying an inspection part performed in step S703 in Fig. 7, step S1102 in Fig. 11, or step S1302 or S1304 in Fig. 13 will be described. In the above embodiments, the descriptions have been given to the operations on the assumption that an inspection part of the entirety of the human body is identified. However, the same applies to a localized inspection target such as a hand.

For example, in an ultrasound inspection for examining a symptom of rheumatism, the joint of the hand or the foot of the human subject is inspected. Thus, in a trained learner used in this variation for predicting the position and orientation of the human body, an inspection target is localized. As the learner, generally, unlike a learner for the entirety of a human body, a learner that outputs skeleton (joint) information regarding a hand or a foot from an input image is used. Alternatively, a learner capable of simultaneously detecting a human body and the skeleton of a hand or a foot may be used.

As a candidate for a localized inspection target, a plurality of candidates such as a hand and a foot is possible as described above. Thus, a plurality of trained learners for detecting pieces of skeleton information regarding parts is prepared corresponding to the parts. Then, when the inspection part identification process is performed, the plurality of prepared learners may be automatically switched according to an external appearance image acquired from the camera 3, or may be switched by selecting and inputting information regarding a corresponding part in advance by an operation of the inspector.

A hand has a thumb and four fingers (the index finger, the middle finger, the ring finger, and the little finger) that each have the first to fourth joints. A trained learner in this variation predicts the position of each joint.

The skeleton information to be output may include the tips of the thumb and four fingers in addition to the joints. As the fourth joint, the same single point is set for all of the thumb and four fingers.

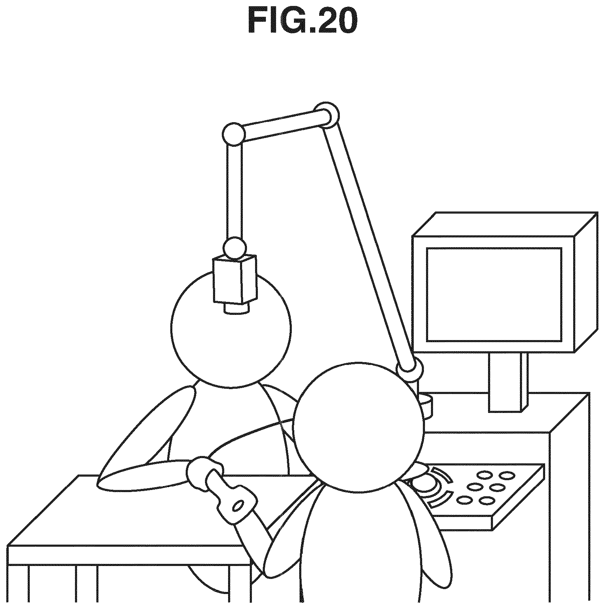

Fig. 20 is an image diagram illustrating the state of an inspection when an inspection target is any of the parts of a hand. The camera 3 is arranged and the position and orientation of the camera 3 are controlled so that the inspection part of the human subject and the ultrasound probe 2 fall within the angle of view of the camera 3.

Fig. 21A is an image diagram in which, in the inspection of a hand as illustrated in this variation, the CPU 7 predicts skeleton information regarding the hand as the prediction process for predicting the position and orientation of the human body and displays on the display 5 the skeleton information in a superimposed manner on an external appearance image from the camera 3.

Fig. 21B is an example where in the inspection of a hand as illustrated in this variation, an image acquired by the camera 3 and the prediction result (cross lines) of the position and orientation of the probe 2 are displayed in a superimposed manner on the display 5.

Fig. 21C is an example where the prediction result of the position and orientation of the human body and the prediction result of the position and orientation of the probe 2 are displayed together on the display 5. By a process similar to that of step S903, it is possible to identify an inspection part, i.e., which joint of which finger.

As described above, in the present embodiment, the position and orientation of a human body is predicted using a learner obtained by machine learning at the time of inspection, and an inspection part is identified by combining this prediction result and the prediction result of the position of an inspection device, whereby it is possible to identify an inspection part with higher accuracy.

In the present embodiment, to predict the position of the inspection device, the inspection device is detected by a rule-based process in which processing load is lower than that in the detection of the position and orientation of the human body. Thus, it is possible to provide an inspection system with less processing load while tracking the position of a probe that moves by a large amount and moves frequently.

### Other Embodiments

The purpose of the present invention can also be achieved as follows. That is, a storage medium recording a program code of software in which a procedure for achieving the functions of the above embodiments is described is supplied to a system or an apparatus. A computer (or a CPU or an MPU) of the system or the apparatus then reads and executes the program code stored in the storage medium.

In this case, the program code itself read from the storage medium achieves the novel functions of the present invention, and the storage medium storing the program code and a program constitute the present invention.

Examples of the storage medium for supplying the program code include a flexible disk, a hard disk, an optical disc, and a magneto-optical disc. Further, a Compact Disc Read-Only Memory (CD-ROM), a Compact Disc-Recordable (CD-R), a Compact Disc-ReWritable (CD-RW), a Digital Versatile Disc Read-Only Memory (DVD-ROM), a Digital Versatile Disc Random-Access Memory (DVD-RAM), a Digital Versatile Disc ReWritable (DVD-RW), a Digital Versatile Disc Recordable (DVD-R), a magnetic tape, a non-volatile memory card, and a ROM can also be used.

The functions of the above embodiments are achieved by making the program code read by the computer executable. Further, the present invention also includes a case where, based on an instruction from the program code, an OS operating on the computer performs a part or all of actual processing, and the functions of the above embodiments are achieved by the processing.

Further, the present invention also includes the following case. First, the program code read from the storage medium is written to a memory included in a function extension board inserted into the computer or a function extension unit connected to the computer. Then, based on an instruction from the program code, a CPU included in the function extension board or the function extension unit performs a part or all of actual processing.

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)^{™}), a flash memory device, a memory card, and the like.

While the present invention has been described with reference to exemplary embodiments, it is to be understood that the invention is not limited to the disclosed exemplary embodiments.

## Claims

1. An information processing apparatus (1) comprising:
acquisition means (15) for acquiring a first image including at least a part of an inspection device (2) captured by image capturing means (3), and a second image including at least a part of a subject captured by the image capturing means (3);
first prediction means (7) for predicting a position of the inspection device (2) with respect to a 3D real space based on the first image;
second prediction means (7) for predicting position and orientation information regarding the subject with respect to the 3D real space based on the second image; and
part identification means (7) for identifying an inspection part of the subject based on the prediction results of the first and second prediction means (7),
wherein, based on a learning model trained in advance using a plurality of images of training data including subjects similar to the subject, the second image is input to the second prediction means, and the second prediction means (7) outputs the position and orientation information regarding the subject,
wherein the part identification means (7) is arranged to identify the inspection part of the subject based on distances from the position of the inspection device (2) predicted by the first prediction means (7) to a plurality of joint points of the subject,
wherein the part identification means (7) is arranged to calculate evaluation values so that greater weight is added to a joint point having a smaller distance from the position of the inspection device (2) predicted by the first prediction means (7) to the joint point, and identifies the inspection part of the subject based on the evaluation value calculated for each of the plurality of joint points,
wherein the second prediction means (7) is arranged to output a plurality of reliability distributions indicating distributions of probabilities of presence of a plurality of parts in an area corresponding to the second image,
wherein the part identification means (7) is further arranged to identify the inspection part of the subject based on the plurality of reliability distributions, and
wherein the second prediction means (7) is arranged to output, as the position and orientation information regarding the subject, skeleton information indicating the plurality of joint points of the subject and positions of the plurality of joint points.

2. The information processing apparatus according to claim 1, wherein the first prediction means (7) is arranged to predict the position of the inspection device (2) by a filtering process and a shape recognition process.

3. The information processing apparatus according to claim 1,
wherein the second prediction means (7) is further arranged to output information regarding a reliability of each of the plurality of joint points of the subject, and
wherein the part identification means (7) is further arranged to identify the inspection part of the subject based on the information regarding the reliability.

4. The information processing apparatus according to claim 1, wherein the part identification means (7) is arranged to determine, as a plurality of joint points of the subject indicating the plurality of parts, positions of peaks of reliabilities in the reliability distributions corresponding to the plurality of parts.

5. The information processing apparatus according to claim 1, wherein the part identification means (7) is further arranged to calculate an evaluation value of each of the plurality of parts based on a distance between the part and the position of the inspection device (2) predicted by the first prediction means (7) and the reliability distributions, and identify the inspection part of the subject based on the evaluation value.

6. The information processing apparatus according to claims 1 to 5, wherein the acquisition means (15) is arranged to acquire the first and second images from an image obtained by capturing an image once.

7. The information processing apparatus according to claims 1 to 6, wherein the first prediction means (7) is arranged to detect the position of the inspection device (2) by detecting a marker disposed on the inspection device (2) from the first image.

8. The information processing apparatus according to claims 1 to 6, wherein the first prediction means (7) is arranged to detect the position and an orientation of the inspection device (2) by detecting a marker disposed on the inspection device (2) from the first image.

9. An inspection system comprising:
one or more processors (7); and
a memory (8, 9) storing instructions which, when the instructions are executed by the one or more processors (7), cause the inspection system to function as the information processing apparatus according to claim 1.

10. A computer-implemented information processing method comprising:
acquiring (S801) a first image including at least a portion of an inspection device captured by an image capturing means (3), and a second image including at least a portion of a subject captured by the image capturing means (3);
predicting (S902) a position of the inspection device (2) with respect to a 3D real space based on the first image;
predicting (S802) position and orientation information regarding the subject with respect to the 3D real space based on the second image; and
identifying (S903) an inspection part of the subject based on results of the predictions,
wherein, based on a learning model trained in advance using a plurality of images of training data including subjects similar to the subject, the second image is input, and the position and orientation information regarding the subject is output,
wherein the inspection part of the subject is identified based on distances from the position of the inspection device (2) to a plurality of joint points of the subject,
wherein the inspection part of the subject is identified by calculating evaluation values so that greater weight is added to a joint point having a smaller distance from the position of the inspection device (2) to the joint point, and identifying the inspection part of the subject based on the evaluation value calculated for each of the plurality of joint points,
wherein a plurality of reliability distributions indicating distributions of probabilities of presence of a plurality of parts in an area corresponding to the second image is outputted, and
wherein the inspection part of the subject is identified based on the plurality of reliability distributions, and
wherein the position and orientation information regarding the subject is output as skeleton information indicating the plurality of joint points of the subject and positions of the plurality of joint points.

11. A computer-readable storage medium comprising instructions which, when executed by a computer, causes the computer to perform the method according to claim 10.

## Patentansprüche

1. Informationsverarbeitungsvorrichtung (1), umfassend:
eine Erfassungseinrichtung (15) zum Erfassen eines durch eine Bildaufnahmeeinrichtung (3) aufgenommenen ersten Bilds, das zumindest einen Teil einer Inspektionsvorrichtung (2) enthält, und eines durch die Bildaufnahmeeinrichtung (3) aufgenommenen zweiten Bildes, das zumindest einen Teil eines Objekts enthält;
eine erste Vorhersageeinrichtung (7) zum Vorhersagen einer Position der Inspektionsvorrichtung (2) in Bezug auf einen realen 3D-Raum basierend auf dem ersten Bild;
eine zweite Vorhersageeinrichtung (7) zum Vorhersagen von Positions- und Orientierungsinformationen bezüglich des Objekts in Bezug auf den realen 3D-Raum basierend auf dem zweiten Bild; und
eine Teilidentifikationseinrichtung (7) zum Identifizieren eines Inspektionsteils des Objekts basierend auf den Vorhersageergebnissen der ersten und zweiten Vorhersageeinrichtung (7),
wobei basierend auf einem Lernmodell, das im Voraus unter Verwendung mehrerer Bilder von Trainingsdaten trainiert wurde, die dem Objekt ähnliche Objekte enthalten, das zweite Bild in die zweite Vorhersageeinrichtung eingegeben wird und die zweite Vorhersageeinrichtung (7) die Positions- und Orientierungsinformationen bezüglich des Objekts ausgibt,
wobei die Teilidentifikationseinrichtung (7) ausgelegt ist, den Inspektionsteil des Objekts basierend auf Abständen von der durch die erste Vorhersageeinrichtung (7) vorhergesagten Position der Inspektionsvorrichtung (2) zu mehreren Verbindungspunkten des Objekts zu identifizieren,
wobei die Teilidentifikationseinrichtung (7) ausgelegt ist, Evaluierungswerte so zu berechnen, dass einem Verbindungspunkt mit einem kleineren Abstand von der durch die erste Vorhersageeinrichtung (7) vorhergesagten Position der Inspektionsvorrichtung (2) zum Verbindungspunkt ein größeres Gewicht hinzugefügt wird, und den Inspektionsteil des Objekts basierend auf dem für einen jeweiligen der mehreren Verbindungspunkte berechneten Bewertungswert identifiziert,
wobei die zweite Vorhersageeinrichtung (7) ausgelegt ist, mehrere Zuverlässigkeitsverteilungen auszugeben, die Verteilungen von Wahrscheinlichkeiten des Vorhandenseins mehrerer Teile in einem dem zweiten Bild entsprechenden Bereich angeben,
wobei die Teilidentifikationseinrichtung (7) ferner ausgelegt ist, basierend auf den mehreren Zuverlässigkeitsverteilungen den Inspektionsteil des Objekts zu identifizieren, und
wobei die zweite Vorhersageeinrichtung (7) ausgelegt ist, als die Positions- und Orientierungsinformationen bezüglich des Objekts Skelettinformationen auszugeben, die die mehreren Verbindungspunkte des Objekts und Positionen der mehreren Verbindungspunkte angeben.

2. Informationsverarbeitungsvorrichtung nach Anspruch 1, wobei die erste Vorhersageeinrichtung (7) ausgelegt ist, durch einen Filterprozess und einen Formerkennungsprozess die Position der Inspektionsvorrichtung (2) vorherzusagen.

3. Informationsverarbeitungsvorrichtung nach Anspruch 1,
wobei die zweite Vorhersageeinrichtung (7) ferner ausgelegt ist, Informationen bezüglich einer Zuverlässigkeit eines jeweiligen der mehreren Verbindungspunkte des Objekts auszugeben, und
wobei die Teilidentifikationseinrichtung (7) ferner ausgelegt ist, basierend auf den Informationen bezüglich der Zuverlässigkeit den Inspektionsteil des Objekts zu identifizieren.

4. Informationsverarbeitungsvorrichtung nach Anspruch 1, wobei die Teilidentifikationseinrichtung (7) ausgelegt ist, als mehrere Verbindungspunkte des Objekts, die die mehreren Teile angeben, Positionen von Zuverlässigkeits-Peaks in den den mehreren Teilen entsprechenden Zuverlässigkeitsverteilungen zu bestimmen.

5. Informationsverarbeitungsvorrichtung nach Anspruch 1, wobei die Teilidentifikationseinrichtung (7) ferner ausgelegt ist, einen Evaluierungswert eines jeweiligen der mehreren Teile basierend auf einem Abstand zwischen dem Teil und der durch die erste Vorhersageeinrichtung (7) vorhergesagten Position der Inspektionsvorrichtung (2) sowie den Zuverlässigkeitsverteilungen zu berechnen, und den Inspektionsteil des Objekts basierend auf dem Bewertungswert zu identifizieren.

6. Informationsverarbeitungsvorrichtung nach den Ansprüchen 1 bis 5, wobei die Erfassungseinrichtung (15) ausgelegt ist, das erste und das zweite Bild aus einem durch einmaliges Aufnehmen eines Bilds erhaltenen Bild zu erfassen.

7. Informationsverarbeitungsvorrichtung nach den Ansprüchen 1 bis 6, wobei die erste Vorhersageeinrichtung (7) ausgelegt ist, die Position der Inspektionsvorrichtung (2) durch Detektieren einer auf der Inspektionsvorrichtung (2) angeordneten Markierung aus dem ersten Bild zu detektieren.

8. Informationsverarbeitungsvorrichtung nach den Ansprüchen 1 bis 6, wobei die erste Vorhersageeinrichtung (7) ausgelegt ist, die Position und eine Ausrichtung der Inspektionsvorrichtung (2) durch Detektieren einer auf der Inspektionsvorrichtung (2) angeordneten Markierung aus dem ersten Bild zu detektieren.

9. Inspektionssystem, umfassend:
einen oder mehrere Prozessoren (7); und
einen Speicher (8, 9), der Anweisungen speichert, die, wenn die Anweisungen durch den einen oder die mehreren Prozessor(en) (7) ausgeführt werden, das Inspektionssystem veranlassen, als die Informationsverarbeitungsvorrichtung nach Anspruch 1 zu funktionieren.

10. Computerimplementiertes Informationsverarbeitungsverfahren, umfassend:
Erfassen (S801) eines durch eine Bildaufnahmeeinrichtung (3) aufgenommenen ersten Bilds, das zumindest einen Abschnitt einer Inspektionsvorrichtung enthält, und eines durch die Bildaufnahmeeinrichtung (3) aufgenommenen zweiten Bilds, das zumindest einen Abschnitt eines Objekts enthält;
Vorhersagen (S902) einer Position der Inspektionsvorrichtung (2) in Bezug auf einen realen 3D-Raum basierend auf dem ersten Bild;
Vorhersagen (S802) von Positions- und Orientierungsinformationen bezüglich des Objekts in Bezug auf den realen 3D-Raum basierend auf dem zweiten Bild; und
Identifizieren (S903) eines Inspektionsteils des Objekts basierend auf Ergebnissen der Vorhersagen,
wobei basierend auf einem Lernmodell, das im Voraus unter Verwendung mehrerer Bilder von Trainingsdaten, die dem Objekt ähnliche Objekte enthalten, trainiert wurde, das zweite Bild eingegeben wird und die Positions- und Orientierungsinformationen bezüglich des Objekts ausgegeben werden,
wobei der Inspektionsteil des Objekts basierend auf Abständen von der Position der Inspektionsvorrichtung (2) zu mehreren Verbindungspunkten des Objekts identifiziert wird,
wobei der Inspektionsteil des Objekts identifiziert wird durch Berechnen von Bewertungswerten so, dass einem Verbindungspunkt mit einem kleineren Abstand von der Position der Inspektionsvorrichtung (2) zum Verbindungspunkt ein größeres Gewicht hinzugefügt wird, und Identifizieren des Inspektionsteils des Objekts basierend auf dem für einen jeweiligen der mehreren Verbindungspunkte berechneten Bewertungswert,
wobei mehrere Zuverlässigkeitsverteilungen ausgegeben werden, die Verteilungen von Wahrscheinlichkeiten des Vorhandenseins mehrerer Teile in einem dem zweiten Bild entsprechenden Bereich angeben, und
wobei der Inspektionsteil des Objekts basierend auf den mehreren Zuverlässigkeitsverteilungen identifiziert wird, und
wobei die Positions- und Orientierungsinformationen bezüglich des Objekts als Skelettinformationen ausgegeben werden, die die mehreren Verbindungspunkte des Objekts und Positionen der mehreren Verbindungspunkte angeben.

11. Computerlesbares Speichermedium, umfassend Anweisungen, die, wenn sie durch einen Computer ausgeführt werden, den Computer veranlassen, das Verfahren nach Anspruch 10 durchzuführen.

## Revendications

1. Appareil de traitement d'informations (1) comprenant :
un moyen d'acquisition (15) pour acquérir une première image comportant au moins une partie d'un dispositif d'inspection (2) pris par un moyen de capture d'image (3), et une deuxième image comportant au moins une partie d'un sujet pris par le moyen de capture d'image (3) ;
un premier moyen de prédiction (7) pour prédire une position du dispositif d'inspection (2) par rapport à un espace réel tridimensionnel basé sur la première image ;
un deuxième moyen de prédiction (7) pour prédire des informations de position et d'orientation concernant le sujet par rapport à l'espace réel tridimensionnel basé sur la deuxième image ; et
un moyen d'identification de partie (7) pour identifier une partie d'inspection du sujet en se basant sur les résultats de prédiction des premier et deuxième moyens de prédiction (7),
dans lequel, en se basant sur un modèle d'apprentissage entraîné à l'avance en utilisant une pluralité d'images de données d'entraînement comportant des sujets similaires au sujet, la deuxième image est introduite dans le deuxième moyen de prédiction, et le deuxième moyen de prédiction (7) délivre en sortie les informations de position et d'orientation concernant le sujet,
dans lequel le moyen d'identification de partie (7) est agencé pour identifier la partie d'inspection du sujet en fonction de distances entre la position du dispositif d'inspection (2) prédite par le premier moyen de prédiction (7) et une pluralité de points de jonction du sujet,
dans lequel le moyen d'identification de partie (7) est agencé pour calculer des valeurs d'évaluation de telle manière qu'un poids plus élevé est ajouté à un point de jonction ayant une petite distance entre la position du dispositif d'inspection (2) prédite par le premier moyen de prédiction (7) et le point de jonction, et identifie la partie d'inspection du sujet d'après la valeur d'évaluation calculée pour chacun des points de la pluralité de points de jonction,
dans lequel le deuxième moyen de prédiction (7) est agencé pour délivrer en sortie une pluralité de distributions de fiabilité indiquant des distributions de probabilités de présence d'une pluralité de parties dans une zone correspondant à la deuxième image,
dans lequel le moyen d'identification de partie (7) est agencé en outre pour identifier la partie d'inspection du sujet d'après la pluralité de distributions de fiabilité, et
dans lequel le deuxième moyen de prédiction (7) est agencé pour délivrer en sortie, comme informations de position et d'orientation concernant le sujet, des informations de squelette indiquant la pluralité de points de jonction du sujet et les positions de la pluralité de points de jonction.

2. Appareil de traitement d'informations selon la revendication 1, dans lequel le premier moyen de prédiction (7) est agencé pour prédire la position du dispositif d'inspection (2) au moyen d'un processus de filtrage et d'un processus de reconnaissance de forme.

3. Appareil de traitement d'informations selon la revendication 1,
dans lequel le deuxième moyen de prédiction (7) est agencé en outre pour délivrer des informations concernant une fiabilité de chacun des points de jonction du sujet, et
dans lequel le moyen d'identification de partie (7) est agencé en outre pour identifier la partie d'inspection du sujet d'après les informations concernant la fiabilité.

4. Appareil de traitement d'informations selon la revendication 1, dans lequel le moyen d'identification de partie (7) est agencé pour déterminer, en tant que pluralité de points de jonction du sujet indiquant la pluralité de parties, des positions de pics de fiabilités dans les distributions de fiabilité correspondant à la pluralité de parties.

5. Appareil de traitement d'informations selon la revendication 1, dans lequel le moyen d'identification de partie (7) est agencé en outre pour calculer une valeur d'évaluation de chacune des parties en fonction d'une distance entre la partie et la position du dispositif d'inspection (2) prédite par le premier moyen de prédiction (7) et des distributions de fiabilité, et pour identifier la partie d'inspection du sujet d'après la valeur d'évaluation.

6. Appareil de traitement d'informations selon les revendications 1 à 5, dans lequel le moyen d'acquisition (15) est agencé pour acquérir les première et deuxième images à partir d'une image obtenue en capturant une image en une fois.

7. Appareil de traitement d'informations selon les revendications 1 à 6, dans lequel le premier moyen de prédiction (7) est agencé pour détecter la position du dispositif d'inspection (2) en détectant un repère placé sur le dispositif d'inspection (2) à partir de la première image.

8. Appareil de traitement d'informations selon les revendications 1 à 6, dans lequel le premier moyen de prédiction (7) est agencé pour détecter la position et une orientation du dispositif d'inspection (2) en détectant un repère placé sur le dispositif d'inspection (2) à partir de la première image.

9. Système d'inspection comprenant :
un ou plusieurs processeurs (7) ; et
une mémoire (8, 9) stockant des instructions qui, lorsque les instructions sont exécutées par lesdits un ou plusieurs processeurs (7), font fonctionner le système d'inspection en tant qu'appareil de traitement d'informations selon la revendication 1.

10. Procédé informatique de traitement d'informations, comprenant les étapes suivantes :
acquérir (S801) une première image comportant au moins une partie d'un dispositif d'inspection pris par un moyen de capture d'image (3), et une deuxième image comportant au moins une partie d'un sujet pris par le moyen de capture d'image (3) ;
prédire (S902) une position du dispositif d'inspection (2) par rapport à un espace réel tridimensionnel basé sur la première image ;
prédire (S802) des informations de position et d'orientation concernant le sujet par rapport à l'espace réel tridimensionnel basé sur la deuxième image ; et
identifier (S903) une partie d'inspection du sujet en se basant sur des résultats des prédictions,
dans lequel, en se basant sur un modèle d'apprentissage entraîné à l'avance en utilisant une pluralité d'images de données d'entraînement comportant des sujets similaires au sujet, la deuxième image est introduite, et les informations de position et d'orientation concernant le sujet sont délivrées en sortie,
dans lequel la partie d'inspection du sujet est identifiée en fonction de distances entre la position du dispositif d'inspection (2) et une pluralité de points de jonction du sujet,
dans lequel la partie d'inspection du sujet est identifiée en calculant des valeurs d'évaluation de telle manière qu'un poids plus élevé est ajouté à un point de jonction ayant une petite distance entre la position du dispositif d'inspection (2) et le point de jonction, et en identifiant la partie d'inspection du sujet d'après la valeur d'évaluation calculée pour chacun des points de la pluralité de points de jonction,
dans lequel une pluralité de distributions de fiabilité indiquant des distributions de probabilités de présence d'une pluralité de parties dans une zone correspondant à la deuxième image est délivrée en sortie, et
dans lequel la partie d'inspection du sujet est identifiée d'après la pluralité de distributions de fiabilité, et
dans lequel les informations de position et d'orientation concernant le sujet sont délivrées sous la forme d'informations de squelette indiquant la pluralité de points de jonction du sujet et les positions de la pluralité de points de jonction.

11. Support de stockage lisible par ordinateur comprenant des instructions qui, lorsqu'elles sont exécutées par un ordinateur, font exécuter par l'ordinateur le procédé de la revendication 10.
